# EUROPÄISCHE PATENTANMELDUNG

(11) **EP 2 671 516 A1**
(43) Veröffentlichungstag der Anmeldung: **11.12.2013**
(21) Anmeldenummer: 13002912.7
(22) Anmeldetag: 06.06.2013
(51) Int. Cl.: A61B 17/04, A61B 17/06

(54) **Fadeneinfuehrvorrichtung**

(30) Priorität: 09.06.2012 DE 102012011488
(71) Anmelder: Rene La Fontaine, 76863 Herxheim (DE); Daehoon Bae, 76751 Jockgrim (DE)
(72) Erfinder: Die Erfindernennung liegt noch nicht vor
(74) Vertreter: Wagner, Jutta

(57) **Zusammenfassung**

Die vorliegende Erfindung betrifft eine Fadeneinführvorrichtung (100) mit einer Nadel (130), die eine Eindringöffnung (131) aufweist, die entlang der Längsrichtung in einer elliptischen Form an einer Schräge geformt ist, und die einen Faden (140) aufweist, der in der Eindringöffnung (131) der Nadel gebildet ist, die an einem Ende der Nadel angeordnet ist, das in den Körper eingeführt werden soll, wobei der Faden (140) an der Eindringöffnung (131) gebogen ist, und die Eindringöffnung (131) zusätzlich mindestens eine Fadeneinführrille (131a) an dem Bereich angrenzend an den Faden (140) aufweist.

## Beschreibung

### TECHNISCHES GEBIET

Die vorliegende Erfindung betrifft eine Fadeneinführvorrichtung zum Einführen eines Fadens in einen Muskel zum Anstoßen der Erzeugung von autogenen Substanzen.

### STAND DER TECHNIK

Autogene Substanzen gibt es in Muskelfasern, aus denen Muskeln im menschlichen Körper aufgebaut sind. Wenn Fremdstoffe in Muskeln gelangen, sammeln sich zudem autogene Substanzen um die Fremdstoffe, um die Muskeln vor den eindringenden Fremdstoffen zu schützen. Obwohl die autogenen Substanzen erzeugt werden, um Muskeln zu schützen, haben sie eine Stärkung der Muskeln zur Folge.

Von diesen autogenen Substanzen sind recht viele Arten vorhanden. Sie stellen Bedingungen für die Genesung von Narben bereit, während sie von den Narbengewebezellen abgegeben werden und als chemische Faktoren dienen, die eine Reihe physiologischer Veränderungen an Geweben bewirken, wenn ein Teil des Muskelgewebes in der Haut mit einer künstlichen Narbe versehen wird. Insbesondere auf der Grundlage von Prinzipien der Bioelektrizität, Piezoelektrizität und allgemeiner Theorien der biologischer Regelung werden die chemischen Faktoren in Wärmeenergie umgewandelt, um kleine Blutgefäße zu erweitern, die Zirkulation von Lymphe zu beschleunigen, so dass Stoffwechselfunktionen stark gesteigert und die Versorgung der betreffenden Bereiche mit Nahrung gefördert, bei Krankheiten erzeugte Stoffe über die Zirkulation von Körperflüssigkeiten abtransportiert, in einigen Geweben gleichzeitig Proteine abgebaut, die Bildung peripherer Nerven gesteigert, und in den Nerven von Blutgefäßen Wirkstoffe zur Senkung des Gehalts von Peptiden und 5-Hydroxylamin im Serum erzeugt werden. Demzufolge werden Informationen für die Narbenbereiche, die regeneriert oder durch Nerven geregelt werden sollen, an das Nervenzentrum geleitet, und die Bildung bestimmter biochemischer Stoffe im Körper wird beschleunigt, um die Immunität im menschlichen Körper durch die aktivierte Zirkulation von Körperflüssigkeiten zu steigern, die mit dem Körper verbundenen Eingeweide zu beeinflussen und auch Muskeln zu stärken.

### VON DER ERFINDUNG ZU LÖSENDE AUFGABEN

Die vorliegende Erfindung soll eine Fadeneinführvorrichtung bereitstellen, die verhindert, dass der Faden reißt, und die verhindert, dass er von einem Muskel abrutscht, während der Faden in den Muskel eingeführt wird, um die Wirkung der Erzeugung von autogenen Substanzen zu verstärken.

### MITTEL ZUM LÖSEN DER AUFGABEN

Eine Fadeneinführvorrichtung gemäß der vorliegenden Erfindung umfasst eine Nadel mit einer Eindringöffnung und einen Faden, der in der Eindringöffnung der Nadel angeordnet ist, wobei die Eindringöffnungan dem Ende der Nadel gebildet ist, wo die Nadel in den Körper eingeführt wird, und dieses Ende zur Ausbildung der Eindringöffnung in Längsrichtung unter Ausbildung einer ellitpischen Form abgeschrägt ist, und der Faden an der Eindringöffnung gebogen ist, und die Eindringöffnung mindestens eine Fadeneinführrille in dem an den Faden angrenzenden Bereich aufweist.

Die Fadeneinführrille kann eine Krümmung aufweisen, die stärker als die auf beiden Seiten ist, die entlang der längeren Achse der elliptischen Form angeordnet sind, um die Eindringöffnung zu bilden.

Die Fadeneinführrille kann zusätzlich von der Eindringöffnung her mehr zur Außenseite ragen.

Die Fadeneinführrille kann zusätzlich in dem Bereich angrenzend an den Faden eine gekrümmte Oberfläche aufweisen.

Der Faden kann zusätzlich in der Fadeneinführrille angeordnet und gebogen sein.

Der Faden kann zusätzlich mehrere Vorsprünge aufweisen, die so geformt sind, dass sie einen Neigungswinkel zur Längsrichtung aufweisen.

Die Vorsprünge können zusätzlich in mindestens eine Richtung abgelegt sein, entweder in die Richtung, in die der Faden eingeführt wird, oder in die entgegengesetzte Richtung.

Die Vorsprünge können zusätzlich mit einem Abstand zwischen ihnen geformt sein.

Die Vorsprünge können zusätzlich in mindestens einer Richtung aus dem Fadenquerschnitt ragen.

An einem Ende der Nadel ist vorzugsweise zusätzlich ein Verbindungsabschnitt geformt, der mit einer Schutzkappe verbunden werden kann, um die Nadel abzudecken.

### WIRKUNG DER ERFINDUNG

Eine Fadeneinführvorrichtung gemäß der vorliegenden Erfindung kann verhindern, dass ein Faden reißt, da sie eine Fadeneinführrille mit einer starken Krümmung an einer Schräge vorsieht, die an einem Ende gebildet ist, wo eine Nadel in den Körper eingeführt wird, und einen Faden an der Fadeneinführrille biegt.

Die Fadeneinführvorrichtung gemäß der vorliegenden Erfindung kann zudem autogene Substanzen um den Faden herum im Körper auslösen, da sie mehrere Vorsprünge außen an einem Faden vorsieht, um zu verhindern, dass der Faden abrutscht, während eine Nadel herausgezogen wird.

### KURZE BESCHREIBUNG DER FIGUREN

Figur 1 ist eine perspektivische Ansicht der Fadeneinführvorrichtung gemäß einer Ausführungsform der vorliegenden Erfindung mit einer vergrößerten Ansicht der Einführöffnung.
Figur 2 ist eine perspektivische Ansicht der Fadeneinführvorrichtung mit einer Schutzkappe.
Figur 3 ist eine vergrößerte Vorderansicht der Eindringöffnung ohne Faden.
Figur 4 ist eine Schnittdarstellung des oberen Teils der Fadeneinführvorrichtung.
Figur 5 ist eine Darstellung von verschiedenen Ausführungsformen des Fadens.
Figur 6 ist eine Schnittdarstellung von verschiedenen Ausführungsformen des Fadens.

### AUSFÜHRLICHE BESCHREIBUNG DER ERFINDUNG

Die bevorzugte Ausführungsform der vorliegenden Erfindung wird nachfolgend bezogen auf Figuren ausführlich beschrieben.

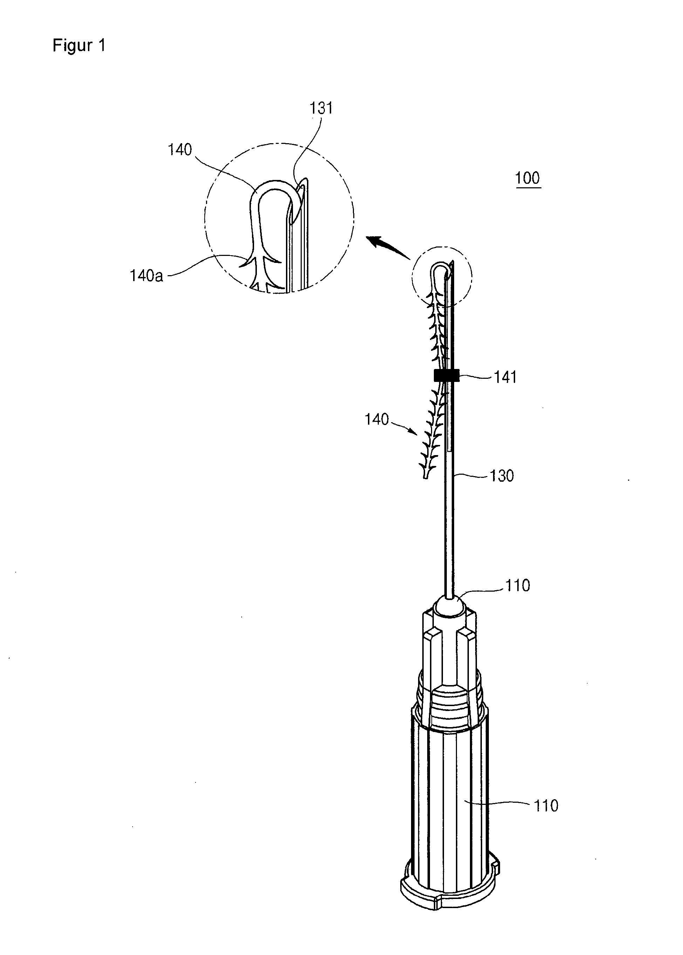

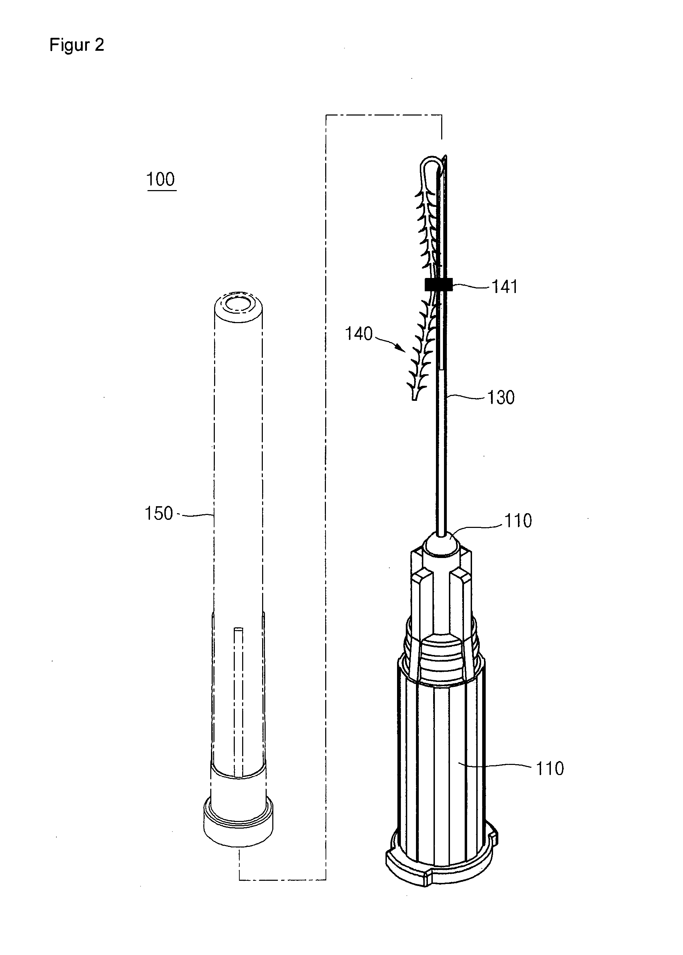

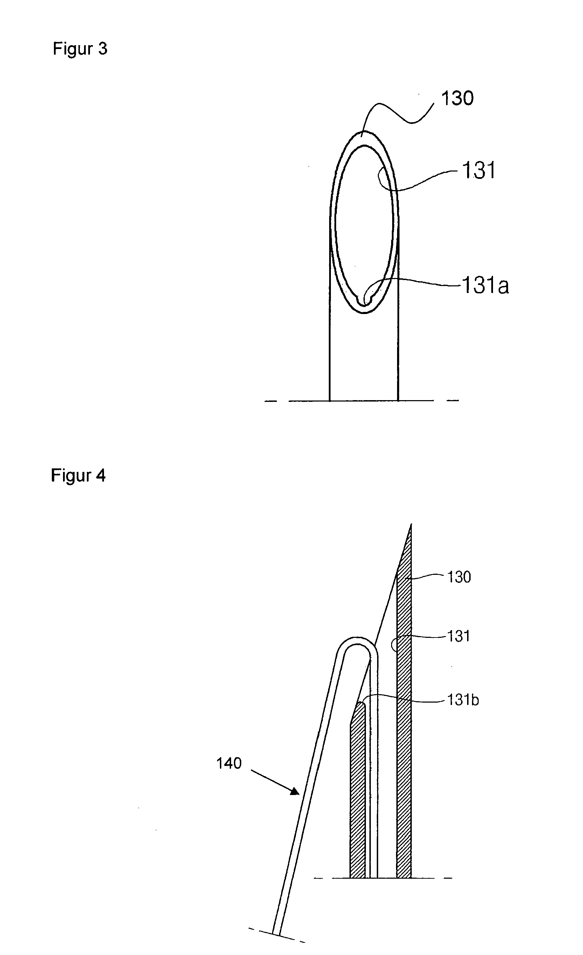

Bezogen auf Figur 1 bis Figur 6 weist die Fadeneinführvorrichtung (100) gemäß der Ausführungsform der vorliegenden Erfindung einen Griff (110), eine Verbindung (120), eine Nadel (130), einen Faden (140) und eine Schutzkappe (150) auf.

Der Griff (110) ermöglicht einem Benutzer, die Fadeneinführvorrichtung (100) zu halten und zu verwenden. Der Griff (110) kann aus einem elastischen Material hergestellt sein, zum einfachen Halten durch den Benutzer, wenn die Fadeneinführvorrichtung (100) verwendet wird. Die Erfindung ist nicht auf die gezeigte Ausgestaltung des Griffes beschränkt, andere Gestaltungen sind möglich und dem Fachmann bekannt

Die Schutzkappe (150) kann die Nadel (130) abdecken und fixieren.

Die Nadel (130) ist mit dem Griff (110) verbunden. Ein Ende der Nadel (130) ist mit dem Griff verbunden und daran befestigt und das andere Ende dringt in die menschliche Haut ein, um in den Körper eingeführt zu werden. Aus diesem Grund ist die Nadel (130) mit einer Schräge an dem anderen Ende versehen, um wie andere Nadeln eingeführt zu werden. Die Nadel (130) kann folglich entlang der Schräge eingeführt werden.

Die Nadel (130) ist mit einer Eindringöffnung (131), die an der Schräge gebildet ist, versehen. Die Eindringöffnung (131) ist entlang der Längsrichtung der Nadel (130) geformt und der Faden (140) ist innerhalb der Eindringöffnung (131) angeordnet.

Die Eindringöffnung (131) ist in einem Kreisabschnitt senkrecht zur Längsrichtung der Nadel (130) geformt. Die Formung der Eindringöffnung (131) entlang der Schräge erfolgt in einer elliptischen Form gemäß der Form der Schräge. Mit anderen Worten besteht die Eindringöffnung (131) aus größeren Abschnitten und kleineren Abschnitten hinsichtlich des Krümmungsradius entsprechend der elliptischen Form. Zusätzlich sind die kleineren Abschnitte des Krümmungsradius an dem Ende der Nadel platziert , das in den Körper eingeführt werden soll, und an dem zweiten Ende gegenüber in der Eindringöffnung (131). Des Weiteren ist der Faden (140) gebogen, wo die Nadel (130) eingeführt wird.

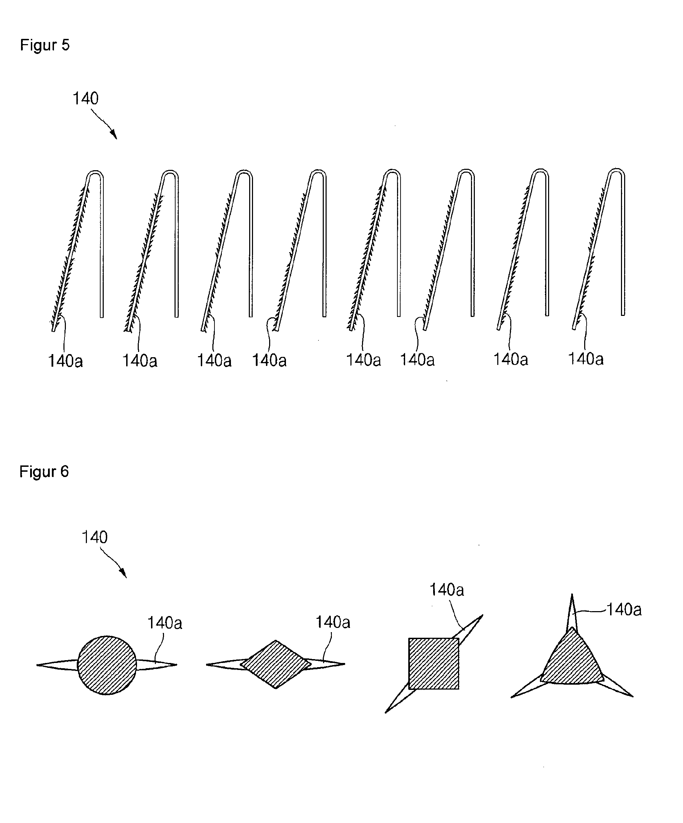

Zudem enthält die Fadeneinführöffnung (131) zusätzlich eine Fadeneinführrille (131a). Die Fadeneinführrille (131 a) befindet sich am zweiten Ende der Fadeneinführöffnung (131). Mit anderen Worten weist die Fadeneinführrille (131 a) einen Krümmungsradius auf, der größer als der der Fadeneinführöffnung (131) am zweiten Ende ist. Die Fadeneinführrille (131 a) ist zudem in der elliptischen Form der Eindringöffnung (131) weiter nach innen geformt. Der Faden (140) ist folglich an der Fadeneinführrille (131 a) angeordnet und grenzt an die Oberfläche der Fadeneinführrille (131 a) an. Da die Fadeneinführrille (131 a) mit einem größeren Krümmungsradius geformt ist als Bereiche mit einem kleineren Krümmungsradius in der Eindringöffnung (131), kann der Faden (140) stabil davor geschützt werden, in dem Kontaktbereich mit dem Faden (140) zerrissen zu werden. Wie in der Schnittdarstellung in Figur 5 dargestellt ist, ist zusätzlich die Fadeneinführungsrille (131a) im Kontakt mit dem Faden (140) in einer Biegung (131 b) verarbeitet, sodass der Faden (140) entlang der Biegung (131 b) an der Fadeneinführrille (131a) angrenzen kann. Der Faden (140) kann folglich in der Fadeneinführrille (131a) aufgenommen sein und stabil in den Körper eingeführt werden, ohne im Biegebereich wegzubrechen.

Der Faden (140) ist in der Eindringöffnung (131) entlang der Längsrichtung der Nadel (130) angeordnet. Der Faden (140) ist an der Fadeneinführrille (131a) angeordnet, die am Ende der Eindringöffnung (131) geformt ist, und er ist an der Fadeneinführrille (131 a) gebogen und erstreckt sich dann in Richtung des Griffs. Der Faden (140) wird entlang der Nadel (130) in den menschlichen Körper eingeführt, in dem fixierten Zustand über den fixierten Block (141) an der Nadel (130). Der fixierte Block (141) wird nicht in den Körper eingeführt, da er in die entgegengesetzte Richtung zur Einführungsrichtung geschoben wird, während die Nadel (130) eingeführt wird, was dazu führt, dass nur die Nadel (130) und der Faden (140) in den Körper eingeführt werden. Der Faden (140) bleibt zudem in dem Körper, selbst nachdem die Nadel (130) zurückgezogen und entfernt ist. Der Faden (140) löst ferner aus, dass autogene Substanzen um ihn herum angesammelt werden, was dazu führt, dass die menschlichen Muskeln gestärkt werden.

Bezogen auf Figur 5 weist der Faden (140) Vorsprünge (140a) an seiner Außenseite auf. Die Vorsprünge (140a) sind in mehreren Stücken angeordnet, die in einem Abstand entlang der Längsrichtung über den gesamten Bereich des Fadens (140), der in den menschlichen Körper eingeführt werden soll geformt sein können. Der Faden (140) kann durch die Vorsprünge (140a) den Kontaktbereich vergrößern, wenn er in den Körper eingeführt wird. Die Vorsprünge (140a) verhindern entsprechend, dass der Faden (140) entlang der Nadel (130) wegrutscht, wenn die Nadel (130) wieder herausgezogen wird, nachdem der Faden (140) in den Körper eingeführt ist. Der Faden (140) kann folglich in dem Körper bleiben, selbst nachdem die Nadel (130) herausgezogen ist. Die Vorsprünge (140a) können zudem mehr regenerative Stoffe für den Menschen auslösen, wenn sie in den Körper eingeführt werden, was zu einer größeren Stärkung menschlicher Muskeln führt.

Die Vorsprünge (140a), die an dem Faden (140) gebildet sind, ragen zudem auf mindestens einer Seite des Fadens (140) entlang der Längsrichtung vor, wie in Figur 5 dargestellt ist. Die Vorsprünge (140a) können ferner zur Einführung des Fadens (140) ausgerichtet sein. Mit anderen Worten können sie in Einführungsrichtung oder in die entgegengesetzte Richtung zur Einführung gelegt sein. Die Vorsprünge (140a) können auch bei einem Faden (140) in verschiedene Richtungen geformt sein. Der Faden (140) kann folglich entsprechend der Richtungen, in die die Vorsprünge (140a) geformt sind, bis zur bevorzugten Tiefe fixiert werden.

Bezogen auf Figur 6 kann der Querschnitt des Fadens (140) auch als verschiedene Formen einschließlich eines Kreises, eines Rhombus, eines Quadrats, eines Dreiecks usw. ausgeführt sein. Die Vorsprünge (140a) ragen von dem Faden (140) aus in mindestens eine Richtung. Wie in Figur 7 dargestellt ist, können die Vorsprünge (140a) von dem Faden (140) aus insbesondere in beide Richtungen vorragen und ihre Querschnittsfläche kann auch verbreitert sein, auch mit dem Vorsprung in drei Richtungen.

Die vorhergehenden Beschreibungen beschreiben lediglich einige Ausführungsformen für die Arbeitsweise der Fadeneinführrichtung der vorliegenden Erfindung. Die vorliegende Erfindung ist nicht auf die zuvor angegebenen Ausführungsformen beschränkt, sondern es kann der technische Gedanke der vorliegenden Erfindung in verschiedenen Änderungen durch jeden, der in dem Gebiet, zu dem die vorliegende Erfindung gehört, über Standardkenntnisse verfügt, innerhalb der Ziele der vorliegenden Erfindung im Umfang der nachfolgenden Patentansprüche geändert werden.

### BEZUGSZEICHENLISTE

- 100:: Fadeneinführvorrichtung
- 110:: Griff
- 130:: Nadel
- 131:: Eindringöffnung
- 131a:: Fadeneinführrille
- 140:: Faden
- 140a:: Vorsprung
- 150:: Schutzkappe

## Patentansprüche

1. Fadeneinführvorrichtung (100) mit einer Nadel (130), die eine Eindringöffnung (131) aufweist, die entlang der Längsrichtung in einer elliptischen Form an einer Schräge geformt ist, und die einen Faden (140) aufweist, der in der Eindringöffnung (131) der Nadel (130) angeordnet ist, wobei die Eindringöffnung (131) an einem Ende der Nadel (130) gebildet ist, das in den Körper eingeführt werden soll, der Faden (140) an der Eindringöffnung (131), die an der Schräge der Nadel (130) geformt ist, gebogen ist, und die Eindringöffnung (131) zusätzlich mindestens eine Fadeneinführrille (131a) an dem Bereich angrenzend an den Faden (140) aufweist.

2. Fadeneinführvorrichtung (100) gemäß Anspruch 1, wobei die Fadeneinführrille (131 a) eine Krümmung aufweist, die stärker als die auf beiden Seiten ist, die entlang der Längsachse einer elliptischen Form angeordnet sind, um die Form der Eindringöffnung (131) zu bilden.

3. Fadeneinführvorrichtung (100) gemäß Anspruch 1 oder 2, wobei die Fadeneinführrille (131a) bezogen auf die Eindringöffnung (131) mehr zur Außenseite ragt.

4. Fadeneinführvorrichtung (100) gemäß Anspruch 1, 2 oder 3, wobei die Fadeneinführrille (131a) in dem an den Faden (140) angrenzenden Bereich eine Krümmung aufweist.

5. Fadeneinführvorrichtung (100) gemäß mindestens einem der Ansprüche 1 bis 4, wobei der Faden (140) in der Fadeneinführrille (131a) angeordnet und gebogen ist.

6. Fadeneinführvorrichtung (100) gemäß mindestens einem der Ansprüche 1 bis 5, wobei der Faden (140) mehrere Vorsprünge (140a) aufweist, die in einem Neigungswinkel zu seiner Längsrichtung geformt sind.

7. Fadeneinführvorrichtung (100) gemäß Anspruch 6, wobei die Vorsprünge (140a) in mindestens eine Richtung gelegt sind, entweder die Richtung, in die der Faden (140) eingeführt ist, oder die entgegengesetzte Richtung.

8. Fadeneinführvorrichtung (100) gemäß Anspruch 6, wobei die Vorsprünge (140a) gegenseitig in einem Abstand zueinander geformt sind.

9. Fadeneinführvorrichtung (100) gemäß Anspruch 6, wobei die Vorsprünge (140a) in mindestens eine Richtung auf dem Fadenquerschnitt ragen.

10. Fadeneinführvorrichtung (100) gemäß mindestens einem der Ansprüche 1 bis 9, wobei ein Ende der Nadel (140) mit einer Verbindung geformt ist, die mit einer Schutzabdeckung (150) kombinierbar ist, um die Nadel (130) abzudecken.
